Europäisches Patentamt

European Patent Office

Office européen des brevets

(19)

(11) Publication number: **0 421 635 B1**

# EUROPEAN PATENT SPECIFICATION

(45) Date of publication of patent specification: **19.07.95**

(51) Int. Cl.6: **A61K 39/295**, C12N 15/62

(21) Application number: **90310264.8**

(22) Date of filing: **19.09.90**

(54) **Chimaeric hepadnavirus core antigen proteins.**

(30) Priority: **19.09.89 GB 8921172**
**13.08.90 GB 9017728**

(43) Date of publication of application:
**10.04.91 Bulletin  91/15**

(45) Publication of the grant of the patent:
**19.07.95 Bulletin  95/29**

(84) Designated Contracting States:
**BE CH DE DK ES FR GB IT LI NL SE**

(56) References cited:
**US-A- 4 859 465**

**NATURE, vol. 330, no. 6146, 26th November -
2nd December 1987, pages 381-384,London,
GB; B.E. CLARKE et al.: "Improved im-
munogenicity of a peptide epitope after fu-
sion to hepatitis B core protein"**

**THE EMBO JOURNAL, vol. 7, no. 3, 1988,
pages 819-824, IRL Press Ltd, Oxford,GB; P.
ARGOS et al.: "A model for the hepatitis B
virus core protein:prediction of antigenic
sites and relationship to RNA virus capsid
proteins"**

(73) Proprietor: **THE WELLCOME FOUNDATION
LIMITED
Unicorn House
160 Euston Road
London NW1 2BP (GB)**

(72) Inventor: **Brown, Alan Louis**
**c/o The Wellcome Foundation Ltd.,
Langley Court
Beckenham,
Kent BR3 3BS (GB)**
Inventor: **Clarke, Berwyn Ewart**
**c/o The Wellcome Foundation Ltd.,
Langley Court
Beckenham,
Kent BR3 3BS (GB)**
Inventor: **Rowlands, David John**
**c/o The Wellcome Foundation Ltd.,
Langley Court
Beckenham,
Kent BR3 3BS (GB)**

(74) Representative: **Stott, Michael John et al
The Wellcome Foundation Limited
Group Patents & Agreements
Langley Court
Beckenham
Kent BR3 3BS (GB)**

EP 0 421 635 B1

PROC. NATL. ACAD. SCI. USA, vol. 86, August 1989, pages 6283-6287; S.J. STAHL et al.: "Immunogenicity of peptide fusions to hepatitis B virus core antigen"

**Description**

This invention relates to the construction of chimaeric hepadnavirus core antigen proteins.

Hepatitis B virus is a hepadnavirus virus with a partly double stranded genome of 3200 nucleotides. The viral DNA is surrounded by the viral coded core antigen (HBcAg) which is enclosed by the similarly coded surface antigen (Robinson, Ann. Rev. Microbiol. 31, 357-377, 1977). Removal of the surface antigen by mild detergent treatment leaves a core particle 27nm in diameter composed of HBcAg and the viral DNA. HBcAg has been expressed in microbial cells as the native polypeptide and as a derivative fused to the terminal eight residues of beta-galactosidase (see Murray et al, EMBO J. 3, 645-650, 1984 for refs).

When synthesized in E. coli the core protein self assembles into 27nm particles which can be visualized under the electron microscope (Cohen and Richmond, Nature, 296, 677-678, 1982) and which are immunogenic in laboratory animals (Stahl et al, Proc. Natl. Acad. Sci. USA 79, 1606-1610, 1982). The amino acid sequence of the core antigen shows a region towards the carboxy terminus which is homologous with that found in protamines (DNA binding proteins). By inference, it has been suggested that this part of the molecule interacts with DNA during assembly of core particles (Pasek et al, Nature, 282, 575-579, 1979).

The use of recombinant particles comprising hepatitis B core antigen and heterologous protein sequences as potent immunogenic moieties is well documented. We have previously shown that addition of heterologous sequences to the amino terminus of the protein results in the spontaneous assembly of particulate structures on the surface of which the heterologous epitope is presented at high density and which are highly immunogenic when inoculated into experimental animals (Clarke et al, Nature 330, 381-384, 1987). Similar results have been reported by other groups using our system (e.g. Chang et al, 2nd International Symposium on positive strand RNA viruses, Vienna, Austria, 1989, abstract 010).

Subsequent experiments by other groups have shown that it is also possible to replace approximately 40 amino acids from the carboxy terminus of the protein with heterologous sequences and still maintain particle morphogenesis (Stahl & Murray, Proc. Natl. Acad. Sci. USA, 86 6283-6287, 1989). Moreover these particles are also immunogenic although, apparently, less so than the amino terminal fusions.

Despite the fact that these fusion particles induce excellent immune responses against the added epitope there still remains room for improvement from several points of view. Firstly, the immune responses against the added epitope, although excellent, do not compare with the parallel responses generated against the HBcAg sequences. Secondly, in both the amino and carboxy terminal fusions the added epitope possesses inherent flexibility because it is only covalently bound at one end. This may be disadvantageous for conformationally rigid epitopes. Thirdly, a model of the structure of hepatitis B core antigen has predicted that, naturally, both the amino and carboxy termini of the protein are located internally within the particles (Argos & Fuller, EMBO J. 1, 819-824, 1988). It is only the inherently hydrophilic nature of most of the heterologous epitopes which directs them to the surface.

We have now located a region of high immunogenicity in a surface region of HBcAg particles. A vector encoding HBcAg and having a restriction site in this region of high immunogenicity has been constructed. Foreign sequences have been inserted at the restriction site, enabling chimaeric HBcAg proteins to be expressed. The chimaeric proteins self-assemble into particles having a foreign epitope exposed on their surface.

Accordingly, the present invention provides particles composed of a chimaeric hepadnavirus core protein having a foreign amino acid sequence comprising an epitope inserted in or replacing all or part of the amino acid sequence from residue 75 to residue 85 in the case where the core antigen is HBcAg or the corresponding amino acid sequence in the case of another hepadnavirus core antigen. HBcAg residues are numbered according to Ono et al, Nucl. Acid. Res. 11, 1747-1757, 1983. Corresponding residues of the core protein of another hepadnavirus may be determined by lining up the sequences of HBcAg and the other core protein.

The chimaeric protein particles can be used to raise antibody specific for the epitope carried by the chimaeric protein. Antibody can therefore be raised in a mammal by administering to the mammal an effective amount of the particles composed of the chimaeric protein wherein the foreign sequence comprises an epitope capable of inducing antibody of the desired specificity. The chimaeric protein particles may be presented for this purpose as a component of a pharmaceutical or veterinary composition also comprising a pharmaceutically or veterinarily acceptable carrier or diluent.

The invention also provides an expression vector which comprises a DNA sequence encoding a hepadnavirus core protein and having (a) a restriction site within the sequence encoding HBcAg amino acid residues 75 to 85 or the corresponding sequence of the core protein of another hepadnavirus or (b) two restriction sites flanking a said sequence or a part of a said sequence encoding HBcAg amino acid residues 68 to 90 or the corresponding sequence of the core protein of another hepadnavirus. Where HBcAg codons 75 to 85 or their counterpart codons for the core protein of another hepadnavirus have been deleted completely or in part, the restriction site is located appropriately in the sequence remaining. Where two restriction sites are provided, typically they are cut by the same restriction enzyme.

Such an expression vector can be provided in a host. The vector provides a starting point for the preparation of a vector capable of expressing the chimaeric protein of the invention. For this purpose, a DNA sequence needs to be constructed which encodes the chimaeric protein. More particularly, a vector is required which incorporates such a DNA sequence and which is capable, when provided in a suitable host, of expressing the chimaeric protein.

A vector capable of expressing the chimaeric protein is prepared by inserting a DNA sequence encoding the foreign sequence into a vector which encodes a hepadnavirus core protein and which has a restriction site or sites (a) or (b) as above. Preferably a restriction site (a) occurs at HBcAg codons 80 and 81 or at the corresponding codons for the core protein of another hepadnavirus. The resulting vector encoding the chimaeric protein is typically provided in a compatible host.

To obtain the chimaeric protein, a host is cultured under such conditions that the chimaeric protein is expressed. The host is provided with an expression vector encoding the chimaeric protein. The chimaeric protein self-assembles into particles when expressed, and can then be isolated. These particles closely resemble the 27 nm core particles composed of HBcAg and viral DNA which can be obtained by denaturing hepatitis B virus. The foreign epitope is exposed on the outer particle surface.

The chimaeric protein comprises a foreign amino acid sequence comprising an epitope. By "foreign" is meant that the sequence is not part of the sequence of the hepadnavirus core protein. The foreign sequence inserted into or replacing all or part of HBcAg amino acid residues 75 to 85 is not therefore part or all of the insert of 39 amino acids near the predicted position of the HBe1 epitope of avian hepatitis viruses, in particular of viruses from ducks (Feitelson and Miller, Proc. Natl. Acad. Sci. USA, 85, 6162-6166, 1988). The hepadnavirus core protein portion of the chimaeric protein is typically a mammalian hepadnavirus core antigen, in particular the human HBcAg or woodchuck WHcAg. Hepatitis B virus adw serotype HBcAg may be used.

Any foreign epitope, i.e. an epitope which is not an epitope of a hepadnavirus core protein, can be presented as part of the chimaeric protein. The epitope is a sequence of amino acid residues capable of raising antibody. The epitope may be an epitope capable of raising neutralising antibody, for example an epitope of an infectious agent or pathogen such as a virus or bacterium. It may be an epitope of a non-infectious agent such as a growth hormone. The foreign sequence may comprise repeats of an epitope, for example up to eight or up to four copies of an epitope. Two copies of an epitope may therefore be present in the foreign sequence. A foreign sequence may comprise two or more different epitopes, for example three or four.

As examples of viruses whose epitopes may be presented there may be mentioned hepatitis A virus, hepatitis B virus, influenza virus, foot-and-mouth disease virus, poliovirus (PV), herpes simplex virus, rabies virus, feline leukaemia virus, human immunodeficiency virus type 1 (HIV-1), HIV-2, simian immunodeficiency virus (SIV), human rhinovirus (HRV), dengue virus and yellow fever virus. The epitope presented by the chimaeric protein may be therefore an epitope of HBsAg, of the pre-S region of HBsAg or of HRV2.

The foreign sequence in the chimaeric protein may be up to 100, for example up to 50, amino acid residues long. The foreign sequence may therefore be up to 40, up to 30, up to 20 or up to 10 amino acid residues in length. The foreign sequence comprises the epitope against which it is desired to induce antibody. The foreign sequence may also comprise further amino acid residues at either or both ends of the epitope.

Where further amino acid residues are present, these may be determined by the manipulations necessary to insert DNA encoding a desired foreign epitope into a vector encoding a hepadnavirus core antigen. They may be the amino acids which naturally flank the epitope. Up to 10, for example up to 4, further amino acids may be provided at either or each end of the foreign epitope.

The foreign sequence is inserted in the sequence of HBcAg residues from 75 to 85 or corresponding residues of another hepadnavirus core protein. Most preferred is to insert the foreign sequence between HBcAg amino acid residues 80 and 81 or between the corresponding residues of another hepadnavirus core protein. Alternatively, all or part of the sequence of core protein residues may be replaced by the foreign sequence. HBcAg amino acid residues 75 to 85 or 80 and 81 or corresponding residues of another hepadnavirus core protein may therefore be replaced by the foreign sequence. Where a foreign sequence replaces all or part of the native core protein sequence, the inserted foreign sequence is generally not shorter than the HBcAg sequence it replaces.

A second foreign amino acid sequence may be fused to the N-terminus or C-terminus of the amino acid sequence of the core protein. This second foreign sequence may also comprise an epitope. This epitope may be identical to or different from the epitope inserted into or replacing all or part of HBcAg amino acid residues 75 to 85 or the corresponding residues of the core protein of another hepadnavirus (the first epitope). Any foreign epitope may be present as the second epitope, as described above in connection with the first epitope. The length and construction of the foreign sequence containing the second epitope may also be as described above in connection with the first epitope.

In order to prepare the chimaeric protein, an expression vector is first constructed. Thus a DNA sequence encoding the desired chimaeric protein is provided. An expression vector is prepared which incorporates the DNA sequence and which is capable of expressing the chimaeric protein when provided in a suitable host. Appropriate transcriptional and translational control elements are provided, including a promoter for the DNA sequence, a transcriptional terminal site, and translational start and stop codons. The DNA sequence is provided in the correct frame such as to enable expression of the polypeptide to occur in a host compatible with the vector.

An appropriate vector capable of expressing the chimaeric protein may be constructed from an HBcAg expression vector having a restriction site (a) or two restriction sites (b) as above.

A DNA sequence encoding the foreign amino acid sequence is inserted into the HBcAg expression vector at the restriction site (a) or in place of the DNA sequence flanked by restriction sites (b). The HBcAg expression vector is digested with the appropriate restriction endonuclease(s) and dephosphorylated. The DNA sequence encoding the foreign sequence is ligated into the cut expression vector. The inserted DNA sequence is typically prepared by standard techniques of oligonucleotide synthesis.

A or each restriction site in the HBcAg expression vector is preferably provided in the HBcAg coding sequence such that the HBcAg amino acid sequence is not altered. A restriction site (a) may occur at HBcAg codons 80 and 81 or the counterpart codons for another hepadnavirus core protein. Preferably, a NheI site is provided in the HBcAg coding sequence at codons 80 and 81. E. coli XL-1 Blue harbouring plasmid pPV-Nhe, which is an HBcAg expression vector provided with such a NheI site, was deposited at the National Collection of Industrial and Marine Bacteria, Aberdeen, GB on 12 September 1989 under accession number NCIMB 40210.

Introduction of a novel restriction site can be achieved in either of two ways. First it may be achieved by replacement of a small restriction fragment coding for this region by a series of synthetic oligonucleotides coding for this region and incorporating the novel restriction site (see Example 1).

Secondly it may be achieved by site directed mutagenesis of the same coding region. This may typically be carried out by initially sub-cloning a restriction fragment representing this region into a vector such as M13mp18 which can produce single stranded DNA. Site directed mutagenesis may then be achieved using specific mismatched synthetic oligonucleotides by standard methods. Such a mutated restriction fragment can then be replaced into the parent gene in a suitable expression vector.

The expression vectors encoding a chimaeric protein are provided in an appropriate host. The chimaeric protein is then expressed. Cells harbouring the vector are grown/cultured so as to enable expression to occur. The chimaeric protein that is expressed self-assembles into particles. The chimaeric particles may then be isolated.

Thus the invention also provides:

- a host transformed with a vector which comprises DNA sequence encoding a chimaeric protein in accordance with the invention, so that the chimaeric protein is able to be expressed therein;
- a process for the preparation of such a host, which comprises transforming a host with a compatible expression vector which comprises a DNA sequence encoding a chimaeric protein in accordance with the invention; and
- a process for the preparation of chimaeric protein particles according to the invention, which comprises culturing a transformed host of the invention under such conditions that the chimaeric protein is expressed therein and recovering particles composed of the chimaeric protein which thus form.

Any appropriate host-vector system may be employed. The vector may be plasmid. In that event, a bacterial or yeast host may be used for example E. coli or S. cerevisiae. Alternatively, the vector may be a viral vector. This may be used to transfect cells of a mammalian cell line, such as Chinese hamster ovary (CHO) cells, in order to cause polypeptide expression.

The chimaeric protein may be used as a vaccine for a human or animal. It may be administered in any appropriate fashion. The choice of whether an oral route or a parenteral route such as sub-cutaneous, intravenous or intramuscular administration is adopted and of the dose depends upon the purpose of the vaccination and whether it is a human or mammal being vaccinated. Similar criteria govern the physiologically acceptable carrier or diluent employed in the vaccine preparation. Conventional formulations, carriers or diluents may be used. Typically, however, the fusion protein is administered in an amount of 1-1000 μg per dose, more preferably from 10-100 μg per dose, by either the oral or the parenteral route.

The following Examples illustrate the invention. In the accompanying drawings:

Figure 1 shows plasmid pBc404. B, E and P denote restriction sites for BamHI, EcoRI and PstI respectively; tac denotes the tac promoter; ori denotes the origin of replication; bla denotes β-lactamase and SD denotes the Shine-Dalgarno sequence.

Figure 2 shows the construction of plasmid pPV-Nhe.

Reference Example: Preparation of HBcAg provided at its N-terminus with a short extension comprising PV1 Mahoney VP1 residues 95 to 104

An expression plasmid pPV404 was prepared from the parent plasmid pBc404 shown in Figure 1. E. coli JM101 harbouring pBc404 was deposited at the National Collection of Industrial and Marine Bacteria, Aberdeen, GB on 9 February 1989 under accession number NCIMB 40111. Synthetic oligonucleotides representing amino acids 95 to 104 of VP1 from PV1 Mahoney were ligated into pBc404 using T4 ligase by standard procedures. This resulted in pPV404. The synthetic oligonucleotides, how they anneal together and the coding sequence of the N-terminal extension are as follows:

```
1. AATTCAGATAATCCAGCTAGTACTACCAACAAAGATAAG (39)

2. GATCCTTATCTTTGTTGGTAGTACTAGCTGGATTATCTG (39)


AATTCAG ATAATCCAGC TAGTACTACC AACAAAGATA AG

    GTC TATTAGGTCG ATCATGATGG TTGTTTCTAT TCCTAGG


   10          20          30          40

ATGAATTCAGATAATCCAGCTAGTACTACCAACAAAGATAAGGATCC.....CORE

   M   N   S   D   N   P   A   S   T   T   N   K   D   K   D
   L_____I L_____I


LINKER                    POLIOVIRUS
```

Example 1: Preparation of plasmid pPV-Nhe

A series of peptides 20 amino acids in length were chemically synthesised. These peptides overlapped each other by 10 amino acids and together represented the whole amino acid sequence of the core protein from hepatitis B virus and serotype. Each of these peptides was used to coat microtitre plates in carbonate coating buffer and was used in an enzyme-linked immunosorbent assay (ELISA) analysis with sera from guinea pigs. The guinea pigs had been inoculated with bacterially-expressed hepatitis B core particles.

One of the peptides, corresponding to amino acids 71 to 90, gave an extremely powerful reaction. This indicated that core particles had elicited antibodies in vivo which reacted with the linear peptide sequence. The sequence corresponded roughly to the reported HBe1 epitope of hepatitis B core particles (Williams and LeBouvier, Bibilotheca Haematologica 42, 71-75, 1976). Our results suggested that the insertion into or replacement of this immunodominant region of HBcAg by a foreign epitope may result in a chimaeric protein displaying enhanced immunogenicity with regard to the foreign epitope. We therefore undertook to insert foreign sequences into the adw core sequence.

The strategy which we followed is shown in Figure 2. The initial plasmid was plasmid pPV404. This plasmid expresses large amounts of chimaeric particles in bacteria which are highly immunogenic in animals. The strategy involved the introduction of a unique NheI restriction site at amino acid positions 80-81 in the core gene. This does not result in an amino acid change in the core protein. The nature of the mutation is shown below:

```
Amino acid     L   E   D   P   A   S   R   D   L

adw            TTG GAA GAT CCA GCA TCC AGG GAT CTA

adw-NheI       TTG GAA GAT CCA GCT AGC AGG GAT CTA

                               NheI
```

Initially plasmid pPV404 was digested with restriction enzymes XbaI and AccIII resulting in two fragments of 3.96 kbp and 340 bp. These fragments were separated by electrophoresis on low melting point agarose, excised and the smaller fragment was then further digested with XhoII resulting in 3 fragments as shown in Figure 2.

Concomitantly two oligonucleotides were synthesised with sequences as shown in Figure 2. These oligonucleotides were annealed and phosphorylated by standard procedures such that they represented a linker sequence with XhoII compatible "sticky ends" and an internal NheI site. These oligonucleotides were then ligated into the 340 bp fragment by standard procedures to replace the 19 bp natural XhoII fragment. This ligated material was then ligated back into the large 3.96 kbp fragment and transformed into E. coli strain XL-1 Blue by standard methods.

The design of this strategy did not exclude the possibility of the natural 19 bp XhoII fragment reinserting itself into the vector. To select for bacteria harbouring plasmids containing the new NheI site, a culture was prepared from all the recombinant clones generated during the transformation. This culture was then used to extract plasmid DNA representing the whole "library" of colonies. After caesium chloride purification this DNA was digested with NheI.

Only those recombinant plasmids carrying the new linker would have been digested in this way resulting in linearisation of this population. Linear DNA was therefore purified from the rest of the undigested plasmid molecules by agarose gel electrophoresis and, after religation, was transformed back into E. coli to generate a pool of NheI positive transformants. Individual clones were analysed by restriction mapping. Those clones which were confirmed to have an inserted NheI site were further characterised by DNA sequencing. The restriction map of one of the resulting clones pPV-Nhe, which possessed the correct sequence, is shown in Figure 2.

As previously stated, the design of the experiment ensured the maintenance of the correct HBcAg amino acid sequence. It was not surprising therefore that expression analysis after induction with isopropyl-beta-D-thiogalactopyranoside (IPTG) confirmed the presence of a high yield of PV-HBcAg particles.

Example 2: Preparation of particles composed of chimaeric HBcAg proteins

The ability of pPV-Nhe to express heterologous sequences was initially assessed by insertion of epitopes from human rhinovirus type 2 (HRV2) and hepatitis B surface antigen (HBsAg). This was achieved by insertion of synthetic oligonucleotides coding for each sequence flanked by NheI cohesive ends into NheI digested and dephosphorylated pPV-Nhe. The sequences inserted at the NheI site of pPV-Nhe are shown below:

## HRV2 VP2

Epitope

A  S  V  K  A  E  T  R  L  N  P  D  L  Q  P  T  E  C  A  S

CTAGCGTTAAAGCGGAAACGCGTTTGAACCCAGATCTGCAACCGACCGAATGCG

    GCAATTTCGCCTTTGCGCAAACTTGGGTCTAGACGTTGGCTGGCTTACGCGATC


## HBsAg 139-147

Epitope

A  S  G  A  C  T  K  P  T  D  G  N  C  A  G  A  S

CTAGCGGTGCATGCACAAAACCTACTGATGGTAACTGCGCAGGTG

    GCCACGTACGTGTTTTGGATGACTACCATTGACGCGTCCACGATC

Plasmids ligated with the synthetic oligonucleotides were transformed into E. coli strain XL-1 Blue. Each new construct was designed so that a diagnostic internal restriction site was present allowing rapid screening of the resulting clones. The internal restriction sites were MluI for HRV2 and SphI for HBsAg. Resulting clones possessing correct restriction sites were cultured to high density in nutrient broth and expression of chimaeric proteins was induced by addition of IPTG to the medium. Following incubation for 6 to 8 hours at 37°C bacterial cells were harvested by centrifugation, lysed by standard procedures and expressed proteins analysed by PAGE, Western blotting and ELISA. The presence of particulate structures was determined by sucrose density gradient centrifugation.

Expression of chimaeric proteins comprising either the HRV2 epitope or the HBsAg was observed. The chimaeric proteins self-assembled into particles. Detailed expression analysis on the HRV2 epitope construct showed that expression levels were very high in bacteria, that particle formation was maintained and that the chimaeric protein reacted with anti-HRV2 sera by Western blotting. ELISA analysis also showed that the HRV2 epitope was exposed on the particle surface.

EXAMPLE 3: Preparation of further particulate chimaeric HBcAg proteins

Further epitopes have been inserted into pPV-Nhe. Synthetic oligonucleotides were ligated together to prepare a DNA fragment encoding the epitope and having cohesive Nhe I ends. The DNA fragment was inserted into Nhe I - digested and dephosphorylated pPV-Nhe. Plasmids ligated with the DNA fragment were transformed into

E. coli strain XL-1 Blue.

Clones were cultured to high density in nutrient broth and expression of chimaeric protein was induced by addition of IPTG to the medium. Following incubation for 6 to 8 hours at 37°C bacterial cells were harvested by centrifugation, lysed by standard procedures and expressed proteins analysed by PAGE, Western blotting and/or ELISA. The presence of particulate structures was determined by sucrose density gradient centrifugation.

Chimaeric proteins presenting the following epitopes were obtained in this way. In each case the epitope was flanked by A-S residues due to the cloning procedure.

1. QE1-amino acid residues 15 to 47 from the Pre-S1 region of hepatitis a virus (HBV). This region is implicated in virus-cell interactions. The sequence was derived from the adw serotype.

(a) Synthetic oligonucleotides

```
              10         20         30         40         50
   CTAGTAACT TAAGTGTGCC AAATCCATTA GGATTTCTGC CAGATCATCA
    ATTGA ATTCACACGG TTTAGGTAAT CCTAAAGACG GTCTAGTAGT

              60         70         80         90        100
   GTTAGATCCA GCATTTGGAG CTAATTCGAC CAATCCAGAT TGGGACTTCA
   CAATCTAGGT CGTAAACCTC GATTAAGCTG GTTAGGTCTA ACCCTGAAGT

             110
   ATCCATGTG
   TAGGTACACG ATC
```

(b) Coding sequence

```
              10         20         30         40         50
   GCTAGTAACTTAAGTGTGCCAAATCCATTAGGATTTCTGCCAGATCATCAGTTAGAT
    A  S  N  L  S  V  P  N  P  L  G  F  L  P  D  H  Q  L  D


   CCAGCATTTGGAGCTAATTCGACCAATCCAGATTGGGACTTCAATCCATGTGCTAG
    P  A  F  G  A  N  S  T  N  P  D  W  D  F  N  P  C  A  S
```

2. QM2-133-144 from Pre-S2 region of HBV. This region is a sequential epitope in HBV and can protect chimpanzees from infection. The sequence was derived from the adw serotype.
   (a) Synthetic oligonucleotides

```
              10         20         30         40
   CTAGTGATC CGCGCGTGCG CGGCTTATAC TTACCGGCGG GAG
    ACTAG GCGCGCACGC GCCGAATATG AATGGCCGCC CTCGATC
```

(b) Coding sequence

```
              10         20         30         40
   GCTAGTGATCCGCGCGTGCGCGGCTTATACTTACCGGCGGGAGCTAGC
    A  S  D  P  R  V  R  G  L  Y  L  P  A  G  A  S
```

3. QE2-120-153 from Pre-S2 region of HBV. This is a larger version of 2. The sequence was derived from the adw serotype.

(a) Synthetic oligonucleotides

```
        10         20        30         40         50
CTAGCATGC AATGGAATAG CACCGCGTTA CACCAAGCTT TGCAGGACCC
  GTACG TTACCTTACT GTGGCGCAAT GTGGTTCGAA ACGTCCTGGG

        60         70        80         90        100
TCGAGTACGT GGCTTATACT TACCGGCGGG AGGATCAAGC AGCGGCACCG
AGCTCATGCA CCGAATATGA ATGGCCGCCC TCCTAGTTCG TCGCCGTGGC

       110
TTAATCCGG
AATTAGGCCG ATC
```

(b) Coding sequence

```
        10         20        30         40         50
GCTAGCATGCAATGGAATAGCACCGCGTTACACCAAGCTTTGCAGGACCCTCGAGT
 A  S  M  Q  W  N  S  T  A  L  H  Q  A  L  Q  D  P  R  V

        60         70        80         90        100
ACGTGGCTTATACTTACCGGCGGGAGGATCAAGCAGCGGCACCGTTAATCC
 R  G  L  Y  L  P  A  G  G  S  S  S  G  T  V  N  P

       110
GGCTAGC
 A  S
```

4. pPD1-110-148, a couplex epitope from HBsAg.
(a) Synthetic oligonucleotides

```
        10         20        30         40         50
CTAGTATTC CTGGGTCAAC GACCACGAGC ACCGGACCAT GCAAGACGTG
  ATAAG GACCCAGTTG CTGGTGCTCG TGGCCTGGTA CGTTCTGCAC

        60         70        80         90        100
TACTACACCA GCACAAGGTA ACTCCAAGTT CCCGAGCTGC TGCTGCACAA
ATGATGTGGT CGTGTTCCAT TGAGGTTCAA GGGCTCGACG ACGACGTGTT

       110        120
AACCTACTGA TGGTAACTGC ACTG
TTGGATGACT ACCATTGACG TGACGATC
```

(b) Coding sequence

```
          10        20        30        40        50
GCTAGTATTCCTGGGTCAACGACCACGAGCACCGGACCATGCAAGACGTGTACTA
 A  S | I  P  G  S  T  T  T  S  T  G  P  C  K  T  C  T

          60        70        80        90       100       110
CACCAGCACAAGGTAACTCCAAGTTCCCGAGCTGCTGCTGCACAAAACCTACTGA
 T  P  A  Q  G  N  S  K  F  P  S  C  C  C  T  K  P  T  D

          120
TGGTAACTGCACTGCTAGC
 G  N  C  T | A  S
```

5. pPA1-VP1 101-110 HAV (Hepatitis A virus)
   (a) Synthetic oligonucleotides

```
                        471
           CTAGCAATTCGAATAACAAGGAGTATACATTTCCGG
               GTTAAGCTTATTGTTCCTCATATGTAAAGGCCGATC
                        472
```

(b) Coding sequence

```
               10        20        30        40
     GCTAGCAATTCGAATAACAAGGAGTATACATTTCCGGCTAGC
      A  S | N  S  N  N  K  E  Y  T  F  P | A  S
```

6. pPA2-VP1 13-24 HAV
   (a) Synthetic oligonucleotides

```
                        473
           CTAGCACTGAACAGAATGTTCCGGATCCTCAGGTTGGAG
               GTGACTTGTCTTACAAGGCCTAGGAGTCCAACCTCGATC
                        474
```

(b) Coding sequence

```
                10        20        30        40
     GCTAGCACTGAACAGAATGTTCCGGATCCTCAGGTTGGAGCTAGC
      A  S | T  E  Q  N  V  P  D  P  Q  V  G | A  S
```

7. pPA3-VP3 61-83 HAV

(a) Synthetic oligonucleotides

```
     475                  466                        476
CTAGCGCAGCACAATTTCCCTTCAATGCAAGCGATTCAGTCGGACAACAGATAAAG
    GCGTCGTGTTAAAGGGAAGTTACGTTCGCTAAGTCAGCCTGTTGTCTATTTC
     478                        469                        477
```

```
GTTATACCTGTGGATCCTG
CAATATGGACACCTAGGACGATC
```

(b) Coding sequence

```
          10        20        30        40        50
GCTAGCGCAGCACAATTTCCCTTCAATGCAAGCGATTCAGTCGGACAACAGATAAA
 A   S   A   A   Q   F   P   F   N   A   S   D   S   V   G   Q   Q   I   K

     60        70        80
GGTTATACCTGTGGATCCTGCTAGC
 V   I   P   V   D   P   A   S
```

8. pPA4-VP1 160-182 HAV

    (a) Synthetic oligonucleotides

```
     491                  492                        493
CTAGCACACCTGTTGGACTAGCAGTAGATACTCCCTGGGTTGAGAAAGAGTCAGCA
    GTGTGGACAACCTGATCGTCATCTATGAGGGACCCAACTCTTTCTCAGTCGT
     496                        495                        494
```

```
CTATCGATTGACTATG
GATAGCTAACTGATACGATC
```

(b) Coding sequence

```
          10        20        30        40        50
GCTAGCACACCTGTTGGACTAGCAGTAGATACTCCCTGGGTTGAGAAAGAGTCA
 A   S   T   P   V   G   L   A   V   D   T   P   W   V   E   K   E   S

        60        70
GCACTATCGATTGACTATGCTAGC
 A   L   S   I   D   Y   A   S
```

9. pPA5-VP2 40-60 HAV

(a) Synthetic oligonucleotides

497                 498

```
CTAGCGTTGAACCTCTACGAACCTCGGTTGACAAACCCGGGTCAAAGAGAACTC
      GCAACTTGGAGATGCTTGGAGCCAACTGTTTGGGCCCAGTTTCTCTTGAG
      |501              |500                    |499
```

```
AAGGTGAGAAAG
TTCCACTCTTTCGATC
```

(b) Coding sequence

```
          10        20        30        40        50
GCTAGCGTTGAACCTCTACGAACCTCGGTTGACAAACCCGGGTCAAAGAGAAC
  A   S   V   E   P   L   R   T   S   V   D   K   P   G   S   K   R   T
```

```
     60        70
TCAAGGTGAGAAAGCTAGC
  Q   G   E   K  A   S
```

10. Amino acids 735-752 from gp41 of HIV-1. This is a potential neutralising epitope for HIV.

(a) Synthetic oligonucleotides

```
      10         20         30         40         50
CTAGCGACC GCCCTGAGGG CATCGAGGAA GAGGGCGGTG AGCGCGATCG
    GCTGG CGGGACTCCC GTAGCTCCTT CTCCCGCCAC TCGCGCTAGC
```

```
     60
TGATCGTTCAG
ACTAGCAAGTCGATC
```

(b) Coding sequence

```
          10         20         30         40         50
GCTAGCGACCGCCCTGAGGGCATCGAGGAAGAGGGCGGTGAGCGCGATCGTGAT
  A   S   D   R   P   E   G   I   E   E   E   G   G   E   R   D   R   D
```

```
     60
CGTTCAGCTAGC
  R   S  A   S
```

11. Epitope from feline leukaemia virus gp70 (197-219) implicated in induction of neutralizing antibodies.

a) Synthetic oligonucleotides

```
       10         20         30         40         50
CTAGTACTA TCACTCCACC ACAGGCCATG GGTCCAAACT TAGTCTTACC
    ATGAT AGTGAGGTGG TGTCCGGTAC CCAGGTTTGA ATCAGAATGG
```

```
          60           70          80
   AGATCAAAAG  CCACCAAGTC  GTCAAG
   TGTAGTTTTC  GGTGGTTCAG  CAGTTCGATC
```

b) coding sequence

```
          10           20          30           40
GCTAGTACTATCACTCCACCACAGGCCATGGGTCCAAACTTA
 A  S  T    I    T    P    P    Q    A    M    G    P    N    L

          50           60          70           80
GTCTTACCAGATCAAAAGCCACCAAGTCGTCAAGCTAGC
 V    L    P    D    Q    K    P    P    S    R    Q  A  S
```

Example 4: Preparation of chimaeric protein having a HRV2 epitope at both the amino terminus and inserted between HBcAg residues 80 and 81

Oligonucleotides coding for the HRV2 VP2 epitope shown in Example 2 were inserted into the pPV-Nhe vector as specified in that Example. The recombinant vector was digested with EcoRI and BamHI. A band of approximately 4.4kb was purified by low melting point agarose gel electrophoresis. Synthetic oligonucleotides representing amino acids 156 to 170 of VP2 from HRV2 were ligated into the recombinant vector using T4 ligase by standard procedures. The synthetic oligonucleotides, how they anneal together and the coding sequence of the N-terminal extension were as follows:

```
1.   AATTCAGTTAAAGCGGAAACGCGTTTG

2.   AACCCAGATCTGCAACCGACCGAATGCCGG

3.   GATCCCGGCATTCGGTCGGTTGCA

4.   GATCTGGGTTCAAACGCGTTTCCGCTTTAACTG

AATTCAGTTAAAGCGGAAACGCGTTTGAACCCAGATCTGCAACCGACCGAATGCCGG
    GTCAATTTCGCCTTTGCGCAAACTTGGGTCTAGACGTTGGCTGGCTTACGGCCTAG
```

```
                                              30
ATG  AAT  TCA  GTT  AAA  GCG  GAA  ACG  CGT  TTG  AAC  CCA  GAT  CTG  CAA
 M    N    S    V    K    A    E    T    R    L    N    P    D    L    Q
      LINKER                                  HRV2
                      60
CCG  ACC  GAA  TGC  CGG  GAT  CC
 P    T    E    C    R    D
```

The resulting plasmid was transformed into E. coli strain XL-1 Blue. Clones were cultured to high density in nutrient broth. Expression of chimaeric protein was achieved as described in Example 2. Expressed proteins were analysed by PAGE, Western blotting and ELISA. The presence of particulate structures was determined by sucrose density gradient centrifugation.

Example 5: Animal tests

Immunisation protocol

Female Dunkin Hartley guinea-pigs weighing about 400g were each inoculated intramuscularly with a 0.5 ml dose of a chimaeric HBcAg protein preparation formulated in incomplete Freund's adjuvant (IFA).

Groups of four animals were inoculated with a specified dose of purified core particles and boosted once at either 56 or 70 days with the same initial dose. Blood samples were taken at 14 day intervals throughout the experiment.

ELISA

Antipeptide, antivirus and anti-particle activity in serum samples was measured by a modification of an indirect or double antibody sandwich ELISA method. In the sandwich ELISA polyclonal antipeptide serum (1:200) was used to capture serial dilutions of particles in PBS and 2% dried milk powder. In the indirect ELISA 2 $\mu$g/ml of peptide, particle or virus were coated directly onto microtitre plates. In each case the plates were washed and incubated with test serum samples. Following incubation at 37C for 1-2 hours plates were rewashed and anti-IgG-peroxidase conjugate was added. After a further hour at 37°C the plates were washed and an enzyme substrate (0.04% o-phenylenediamine and 0.004% hydrogen peroxide in 0.1M phosphate 0.05M citrate buffer) was added. The resulting colour development was stopped with sulphuric acid and the A492 was measured in a Titertek Multiskan (Trade Mark, Flow Labs).

The A492 values obtained from dilutions of post-inoculation samples were plotted against the log10 reciprocal antiserum dilution and the antibody titre was calculated by reference to a negative standard (a 1:10 dilution of pre-inoculation serum).

Results

1. Guinea pigs were inoculated intramuscularly with 20 $\mu$g or 2 $\mu$g of particles obtained in Example 3.1 composed of the PreS1-HBcAg chimaeric protein. Bleeds were taken at regular intervals. In each case a peptide composed of the PreS1 insert in the chimaeric HBcAg protein (392), HBcAg with no insert (control) and PreS1-HBcAg particles were coated onto enzyme-linked immunosorbent assay (ELISA) dishes and then assayed against dilutions of the antisera collected. The results are shown in Table 1. Very high levels of antipeptide, antiHBcAg and antiPreS1-HBcAg particles antibody were achieved.

2. The procedure was the same as in 1 except:
   - guinea pigs were inoculated with particles obtained in Example 3.2 composed of the small PreS2 epitope-HBcAg chimaeric protein;
   - a peptide composed of the PreS2 insert (393), HBcAg, PreS2-HBcAg particles and yeast-derived HBsAg particles with PreS2 epitopes incorporated were coated onto ELISA dishes.

   The results are shown in Table 2. Very high levels of antibody were again induced in the guinea pigs.

3. The immune responses induced in guinea pigs and rabbits by particles composed of a chimaeric HBcAg protein in which a HRV2 VP2 epitope is inserted in accordance with the invention ("insert", Example 2) and by particles composed of a chimaeric HBcAg protein in which the same HRV2 VP2 epitope is fused to the amino terminus of HBcAg ("terminal") were compared. The "terminal" chimaeric HBcAg protein was prepared in accordance with the procedure described in JP-A-196299/88. The results are shown in Tables 3 and 4 and are ELISA endpoint titres ($\log_{10}$). The ELISA plates were coated with either a peptide composed of the HRV2 VP2 epitope or HBV. The "insert" chimaeric HBcAg protein gave superior results: higher anti-HRV peptide titres and lower anti-HBV titres.

4. The neutralising antibody responses were looked at of the guinea pigs and rabbits of 3. above. In particular, the responses of the animals to two inoculations of the "insert" particles were assayed. The results are shown in Table 5.

5. Rabbits and guinea pigs were inoculated intramuscularly with particles composed of the chimaeric HBsAg 139-147 epitope-HBcAg protein of Example 2. Anti-peptide 139-147 ($\alpha$ pep 448) and anti-HBsAg ($\alpha$HBsAg) responses were determined using three doses of particles. The results are shown in Table 6. The data shown are titres ($\log_{10}$) prior to a first booster inoculation at 42 days and at the final bleeds day 98. In terms of anti-HBsAg activity, good antibody levels were observed in final bleeds in rabbits and, particularly, in guinea pigs.

15

TABLE 1

| Group 1 | 392 (2μg/ml) | HBcAg (2μg/ml) | PreS1-HBcAg (2μg/ml) |
|---|---|---|---|
| 20μg 14 | 3.57 | 2.89 | 3.78 |
| 28 | 4.48 | 3.73 | 4.28 |
| 42 | 4.54 | 4.16 | 4.59 |
| 71* | 4.63 | 3.87 | 3.79 |
| 77 | 5.39 | 4.20 | 4.44 |
| 84 | 4.69 | 4.24 | 4.46 |
| 98* | 5.15 | 4.32 | 4.73 |
| **Group 2** | | | |
| 2μg 14 | 2.73 | 3.15 | 3.30 |
| 28* | 4.20 | 3.69 | 3.57 |
| 42 | 4.22 | 3.39 | 3.39 |
| 71* | 4.16 | 3.77 | 3.93 |
| 77 | 4.02 | 3.77 | 3.94 |
| 84 | 4.15 | 3.95 | 3.90 |
| 98* | 4.60 | 4.41 | 4.31 |
| ELISA end point titres $\log_{10}$ | | | |

\* mean of group of individuals

## Table 2: Response of guinea pigs to preS2 (QM2/PE3) cores (small epitope)

### (a) 20μg dose

| Days post primary inoculation | Test antigen | | | |
|---|---|---|---|---|
| | peptide 393 | HBcAg | QM2 cores | HBsAg + preS2 |
| 0** | <1* | <1 | <1 | <1 |
| 14 | 2.5 | 2.2 | 3.3 | 2.1 |
| 28 | 3.2 | 3.0 | 4.1 | 3.1 |
| 42 | 3.6 | 3.3 | 4.2 | 3.4 |
| 70** | 3.6 | 4.4 | 4.5 | 3.8 |
| 77 | 3.9 | 4.4 | 4.5 | 3.9 |
| 84 | 4.2 | 4.7 | 5.2 | 4.4 |
| 98 | 4.1 | 4.6 | 4.9 | 4.1 |

16

(b) <u>2μg dose</u>

| Drugs post primary inoculation | Test antigen | | | |
|---|---|---|---|---|
| | peptide <u>393</u> | HBcAg | QM2 <u>cores</u> | HBsAg <u>+preS2</u> |
| 0** | <1* | <1 | <1 | <1 |
| 14 | 2.0 | 2.0 | 3.0 | 1.9 |
| 28 | 2.1 | 2.6 | 3.5 | 2.2 |
| 42 | 2.3 | 3.3 | 3.3 | 2.3 |
| 70** | 2.5 | 3.7 | 3.7 | 3.0 |
| 77 | 3.2 | 4.2 | 4.1 | 3.5 |
| 84 | 3.7 | 4.8 | 4.7 | 4.0 |
| 98 | 3.4 | 4.6 | 4.9 | 4.2 |

* $\log_{10}$ end point titre

** inoculations

Table 3: Comparative immunogenicity of "insert" and "terminal" HBcAg/HRV peptide fusion protein particles in guinea pigs

(a)    Anti-HRV peptide

| Peptide dose (μg) | 28 days post primary | | 56 days post primary | | 28 days post boost | |
|---|---|---|---|---|---|---|
| | insert | terminal | insert | terminal | insert | terminal |
| 1.5 | 3.6* | 2.7 | 4.1 | 2.9 | 4.8 | 3.6 |
| 0.15 | 2.5 | 1.3 | 3.1 | 2.3 | 4.3 | 2.9 |
| 0.015 | 1.9 | <1.0 | 2.1 | <1.0 | 3.5 | <1.0 |

(b)    Anti-HRV

| Peptide dose (μg) | 28 days post primary | | 56 days post primary | | 28 days post boost | |
|---|---|---|---|---|---|---|
| | insert | terminal | insert | terminal | insert | terminal |
| 1.5 | 3.9* | 1.6 | 4.2 | 1.5 | 5.1 | 2.8 |
| 0.15 | 2.3 | 1.2 | 2.5 | 1.6 | 4.2 | 2.5 |
| 0.015 | 1.6 | 1.2 | 1.8 | 1.0 | 3.5 | 1.0 |

* ELISA endpoint  titre ($\log_{10}$)

## Table 4: Comparative immunogenicity of "insert" and "terminal" HBcAg/HRV peptide fusion protein particles in rabbits

(a)  Anti-HRV peptide

| Peptide dose (µg) | 28 days post primary | | 56 days post primary | | 28 days post boost | |
|---|---|---|---|---|---|---|
| | insert | terminal | insert | terminal | insert | terminal |
| 1.5 | 3.0* | 2.2 | 3.2 | 2.4 | 4.2 | 2.9 |
| 0.15 | 2.7 | 1.5 | 2.4 | 1.5 | 3.1 | 1.7 |
| 0.015 | 2.1 | N.D. | 1.8 | N.D. | 2.7 | N.D. |

(b)  Anti- HRV

| Peptide dose (µg) | 28 days post primary | | 56 days post primary | | 28 days post boost | |
|---|---|---|---|---|---|---|
| | insert | terminal | insert | terminal | insert | terminal |
| 1.5 | 2.1* | 1.2 | 2.5 | 1.1 | 4.0 | 1.4 |
| 0.15 | 1.8 | 1.1 | 2.0 | 1.1 | 2.8 | 1.5 |
| 0.015 | 1.7 | N.D. | 1.6 | N.D. | 2.4 | N.D. |

N.D. = not determined

* ELISA endpoint titre ($\log_{10}$)

TABLE 5

| (a) Guinea pigs | Neutralization titre |
|---|---|
| peptide dose (μg) | 90% Plaque reduction |
| 1.5<br>0.15<br>0.015 | 90, 30, 50, 25<br>20, 5<br><5, <5 |
| (b) Rabbits | Neutralization titre |
| peptide dose (μg) | 90% Plaque reduction |
| 1.5<br>0.15<br>0.015 | 300, 5<br>10, 15<br><5, <5 |

TABLE 6

| | | | | Ist Inoc. | 2nd Inoc. |
|---|---|---|---|---|---|
| chimaeric HBsAg 139-147 HBcAg protein | Rabbits | 20μg | αpep448<br>αHBsAg | 3.3<br>1.6 | 3.4<br>2.7 |
| | | 2μg | αpep448<br>αHBsAg | 2.6<br>1.3 | 3.0<br>2.4 |
| | | 0.2μg | αpep448<br>αHBsAg | 2.7<br>1.2 | 3.2<br>1.4 |
| | Guinea Pigs | 20μg | αpep448<br>αHBsAg | 3.9<br>3.6 | 5.1<br>5.0 |
| | | 2μg | αpep448<br>αHBsAg | 3.0<br>1.9 | 4.3<br>4.0 |
| | | 0.2μg | αpep448<br>αHBsAg | 2.1<br><1 | 4.0<br>2.4 |

**Claims**

**Claims for the following Contracting States : BE, CH, DE, DK, FR, GB, IT, LI, NL, SE**

1. Particles composed of a chimaeric hepadnavirus core antigen protein having a foreign amino acid sequence comprising an epitope inserted in or replacing all or part of the amino acid sequence from residue 75 to residue 85 in the case where the core antigen is hepatitis B core antigen (HBcAg) or the corresponding amino acid sequence in the case of the core antigen of another hepadnavirus.

2. Particles according to claim 1, wherein the foreign amino acid sequence is up to 40 amino acid residues in length.

3. Particles according to claim 2, wherein the foreign amino acid sequence is up to 20 amino acid residues in length.

4. Particles according to any one of the preceding claims, wherein the foreign amino acid sequence is inserted between HBcAg residues 80 and 81 or between the corresponding residues of the core protein of another hepadnavirus.

5. Particles according to any one of the preceding claims, wherein the epitope is the epitope of a pathogen.

6. Particles according to claim 5, wherein the epitope is an epitope of hepatitis A virus, hepatitis B virus, influenza virus, foot-and-mouth disease virus, poliovirus, herpes simplex virus, rabies virus, feline leukaemia virus, human immunodeficiency virus type 1 or 2, simian immunodeficiency virus, human rhinovirus, dengue virus or yellow fever virus.

7. Particles according to any one of the preceding claims, wherein a second said foreign amino acid sequence is fused to the N-terminus of the amino acid sequence of the core protein.

8. A vector which comprises a DNA sequence encoding a chimaeric protein as specified in any one of the preceding claims and which is capable, when provided in a suitable host, of expressing the chimaeric protein.

9. A host transformed with a vector according to claim 8 so that the chimaeric protein is able to be expressed therein.

10. A process for the preparation of particles as claimed in any one of claims 1 to 7, which process comprises culturing a host according to claim 9 under such conditions that the chimaeric protein is expressed therein and recovering particles composed of the chimaeric protein which thus form.

11. A process for the preparation of a host as claimed in claim 9, which process comprises transforming a host with a compatible expression vector according to claim 8.

12. An expression vector which comprises a DNA sequence encoding a hepadnavirus core antigen and having (a) a restriction site within the sequence encoding HBcAg amino acid residues 75 to 85 or the corresponding sequence of the core protein of another hepadnavirus or (b) two restriction sites flanking a said sequence or a part of a said sequence.

13. A vector according to claim 12, wherein the restriction site (a) occurs at HBcAg codons 80 and 81 or at the corresponding core protein codons of another hepadnavirus.

14. A vector according to claim 12, which is pPV-Nhe (as harboured in deposit NCIMB 40210).

15. A process for the preparation of a vector as claimed in claim 8 which process comprises:
(i) digesting with the appropriate restriction endonuclease(s) an expression vector as claimed in any one of claims 12 to 14 such as to cut the vector at restriction site (a) or restriction site (b);
(ii) dephosphorylating the digested vector; and
(iii) ligating a DNA sequence encoding a foreign amino acid sequence comprising an epitope into the vector.

16. A pharmaceutical or veterinary formulation comprising a pharmaceutically or veterinarily acceptable carrier or diluent and, as active ingredient, particles as claimed in any one of claims 1 to 7.

**Claims for the following Contracting State : ES**

1. A process for the preparation of particles composed of a chimaeric hepadnavirus core antigen protein having a foreign amino acid sequence comprising an epitope inserted in or replacing all or part of the amino acid sequence from residue 75 to residue 85 in the case where the core antigen is hepatitis B core antigen (HBcAg) or the corresponding amino acid sequence in the case of the core antigen of another hepadnavirus, which process comprises
(I) culturing a host transformed with a compatible expression vector which comprises a DNA sequence encoding a said chimaeric protein under such conditions that the chimaeric protein is expressed in the host, and
(II) recovering particles composed of the chimaeric protein which thus form.

2. A process according to claim 1, wherein the foreign amino acid sequence is up to 40 amino acid residues in length.

**3.** A process according to claim 2, wherein the foreign amino acid sequence is up to 20 amino acid residues in length.

**4.** A process according to any one of the preceding claims, wherein the foreign amino acid sequence is inserted between HBcAg residues 80 and 81 or between the corresponding residues of the core protein of another hepadnavirus.

**5.** A process according to any one of the preceding claims, wherein the epitope is the epitope of a pathogen.

**6.** A process according to claim 5, wherein the epitope is an epitope of hepatitis A virus, hepatitis B virus, influenza virus, foot-and-mouth disease virus, poliovirus, herpes simplex virus, rabies virus, feline leukaemia virus, human immunodeficiency virus type 1 or 2, simian immunodeficiency virus, human rhinovirus, dengue virus or yellow fever virus.

**7.** A process according to any one of the preceding claims, wherein a second said foreign amino acid sequence is fused to the N-terminus of the amino acid sequence of the core protein.

**8.** A process for the preparation of a transformed host which process comprises transforming a host with a compatible expression vector which comprises a DNA sequence encoding a chimaeric hepadnavirus core antigen protein as defined in any one of the preceding claims.

**9.** A process for the preparation of a vector which comprises a DNA sequence encoding a chimaeric hepadnavirus core antigen protein as defined in any one of claims 1 to 7 and which is capable, when provided in a suitable host, of expressing the chimaeric protein; which process comprises:
(i) digesting with the appropriate restriction endonuclease(s) an expression vector which comprises a DNA sequence encoding a hepadnavirus core antigen and having (a) a restriction site within the sequence encoding HBcAg amino acid residues 75 to 85 or the corresponding sequence of the core protein of another hepadnavirus or (b) two restriction sites flanking a said sequence or a part of a said sequence, such as to cut the vector at restriction site (a) or restriction site (b);
(ii) dephosphorylating the digested vector; and
(iii) ligating a DNA sequence encoding a foreign amino acid sequence comprising an epitope into the vector.

**10.** A process according to claim 9, wherein the restriction site (a) occurs at HBcAg codons 80 and 81 or at the corresponding core protein codons of another hepadnavirus.

**11.** A process according to claim 10, wherein the expression vector is pPV-Nhe (as harboured in deposit NCIMB 40210).

**Patentansprüche**

**Patentansprüche für folgende Vertragsstaaten : BE, CH, DE, DK, FR, GB, IT, LI, NL, SE**

**1.** Teilchen bestehend aus chimärischem Hepadnavirus-Kernantigenprotein, das eine fremde Aminosäuresequenz hat, welche ein Epitop enthält, das in die Aminosäuresequenz zwischen Rest 75 und Rest 85 für den Fall, daß das Kernantigen Hepatitis B-Kernantigen (HBcAg) ist, oder in die entsprechende Aminosäuresequenz für den Fall, daß das Kernantigen von einem anderen Hepadnavirus ist, eingefügt ist oder diese ganz oder teilweise ersetzt.

**2.** Teilchen nach Anspruch 1, in denen die fremde Aminosäuresequenz eine Länge von bis zu 40 Aminosäureresten hat.

**3.** Teilchen nach Anspruch 2, in denen die fremde Aminosäuresequenz eine Länge von bis zu 20 Aminosäureresten hat.

**4.** Teilchen nach einem der vorangehenden Ansprüche, in denen die fremde Aminosäuresequenz zwischen die HBcAg-Reste 80 und 81 oder zwischen die entsprechenden Reste des Kernproteins eines anderen Hepadnavirus eingefügt ist.

22

5. Teilchen nach einem der vorangehenden Ansprüche, in denen das Epitop das Epitop eines Krankheitserregers ist.

6. Teilchen nach Anspruch 5, in denen das Epitop ein Epitop des Hepatitis A-Virus, Hepatitis B-Virus, Influenza-Virus, Virus der Maul- und Klauenseuche, Poliovirus, Herpes simplex-Virus, Tollwutvirus, Katzenleukämie-Virus, humanen Immundefizienzvirus Typ 1 oder 2, Affen-Immundefizienzvirus, humanen Rhinovirus, Dengue-Virus oder Gelbfiebervirus ist.

7. Teilchen nach einem der vorangehenden Ansprüche, in denen eine zweite fremde Aminosäuresequenz an das N-Ende der Aminosäuresequenz des Kernproteins kondensiert ist.

8. Vektor, der eine DNA-Sequenz enthält, die ein chimärisches Protein wie es in einem der vorangehenden Ansprüche spezifiziert ist, kodiert und der, wenn er in einem geeigneten Wirt bereitgestellt wird, das chimärische Protein exprimiert.

9. Wirt, der mit einem Vektor gemäß Anspruch 8 transformiert ist, so daß das chimärische Protein darin exprimiert werden kann.

10. Verfahren zur Herstellung von Teilchen, wie sie in einem der Ansprüche 1 bis 7 beansprucht sind; wobei das Verfahren Kultivieren eines Wirts nach Anspruch 9 unter solchen Bedingungen, daß das chimärische Protein darin exprimiert wird, und Isolieren von Teilchen, die aus dem auf diese Weise gebildeten chimärischen Protein bestehen, umfaßt.

11. Verfahren zur Herstellung eines Wirts nach Anspruch 9, wobei das Verfahren ein Transformieren eines Wirts mit einem verträglichen Expressionsvektor nach Anspruch 8 umfaßt.

12. Expressionsvektor, der eine DNA-Sequenz enthält, welche ein Hepadnavirus-Kernantigen kodiert und (a) eine Restriktionsstelle innerhalb der Sequenz, die die HBcAg-Aminosäurereste 75 bis 85 oder die entsprechende Sequenz des Kernproteins eines anderen Hepadnavirus kodiert, oder (b) zwei Restriktionsstellen hat, die diese Sequenz oder einen Teil dieser Sequenz flankieren.

13. Vektor nach Anspruch 12, in dem die Restriktionsstelle (a) an den HBcAg-Kodons 80 und 81 oder an den entsprechenden Kernprotein-Kodons eines anderen Hepadnavirus auftritt.

14. Vektor nach Anspruch 12, der pPV-Nhe (wie in der Hinterlegung NCJMB 40210 verborgen) ist.

15. Verfahren zur Herstellung eines Vektors, wie er in Anspruch 8 beansprucht ist, wobei das Verfahren
(i) Aufschließen eines Expressionsvektors, wie er in einem der Ansprüche 12 bis 14 beansprucht ist, mit der geeigneten Restriktions-Endonuclease (geeigneten Endonucleasen) derart, daß der Vektor an Restriktionsstelle (a) oder Restriktionsstelle (b) geschnitten wird;
(ii) Dephosphorylierung des aufgeschlossenen Vektors;
(iii) Binden einer DNA-Sequenz, die eine fremde Aminosäuresequenz, welche ein Epitop enthält, kodiert, an den Vektor.

16. Pharmazeutische oder tiermedizinische Formulierung, die einen pharmazeutisch oder tiermedizinisch akzeptablen Trägerstoff oder ein pharmazeutisch oder tiermedizinisch akzeptables Verdünnungsmittel und als aktives Ingrediens Teilchen, wie sie in einem der Ansprüche 1 bis 7 beansprucht sind, enthält.

**Patentansprüche für folgenden Vertragsstaat : ES**

1. Verfahren zur Herstellung von Teilchen bestehend aus chimärischem Hepadnavirus-Kernantigenprotein, das eine fremde Aminosäuresequenz hat, welche ein Epitop enthält, das in die Aminosäuresequenz zwischen Rest 75 und Rest 85 für den Fall, daß das Kernantigen Hepatitis B-Kernantigen (HBcAg) ist, oder in die entsprechende Aminosäuresequenz für den Fall, daß das Kernantigen von einem anderen Hepadnavirus ist, eingefügt ist oder diese ganz oder teilweise ersetzt, wobei das Verfahren
(I) Kultivieren eines Wirts, der mit einem verträglichen Expressionsvektor, welcher eine DNA-Sequenz enthält, die das chimärische Protein kodiert, transformiert ist, unter solchen Bedingungen, daß das chimärische Protein in dem Wirt exprimiert wird; und

(II) Isolieren von Teilchen, die aus dem auf diese Weise gebildeten chimärischen Protein bestehen, umfaßt.

2. Verfahren nach Anspruch 1, in denen die fremde Aminosäuresequenz eine Länge von bis zu 40 Aminosäureresten hat.

3. Verfahren nach Anspruch 2, in denen die fremde Aminosäuresequenz eine Länge von bis zu 20 Aminosäureresten hat.

4. Verfahren nach einem der vorangehenden Ansprüche, in denen die fremde Aminosäuresequenz zwischen die HBcAg-Reste 80 und 81 oder zwischen die entsprechenden Reste des Kernproteins eines anderen Hepadnavirus eingefügt ist.

5. Verfahren nach einem der vorangehenden Ansprüche, in denen das Epitop das Epitop eines Krankheitserregers ist.

6. Verfahren nach Anspruch 5, in denen das Epitop ein Epitop des Hepatitis A-Virus, Hepatitis B-Virus, Influenza-Virus, Virus der Maul- und Klauenseuche, Poliovirus, Herpes simplex-Virus, Tollwutvirus, Katzenleukämie-Virus, humanen Immundefizienzvirus Typ 1 oder 2, Affen-Immundefizienzvirus, humanen Rhinovirus, Dengue-Virus oder Gelbfiebervirus ist.

7. Verfahren nach einem der vorangehenden Ansprüche, in denen eine zweite fremde Aminosäuresequenz an das N-Ende der Aminosäuresequenz des Kernproteins kondensiert ist.

8. Verfahren zur Herstellung eines transformierten Wirts, das Transformieren eines Wirts mit einem verträglichen Expressionsvektor, der eine DNA-Sequenz enthält, die ein chimärisches Hepadnavirus-Kernantigen, wie es in einem der vorangehenden Ansprüchen definiert ist, kodiert, umfaßt.

9. Verfahren zur Herstellung eines Vektors, der eine DNA-Sequenz enthält, die ein chimärisches Hepadnavirus-Kernantigenprotein, wie es in einem der Ansprüche 1 bis 7 definiert ist, kodiert, und der, wenn er in einem geeigneten Wirt angesiedelt wird, das chimärische Protein exprimieren kann;
(i) Aufschließen eines Expressionsvektors, der eine DNA-Sequenz enthält, die ein Hepadnavirus-Kernantigen kodiert und die eine Restriktionsstelle (a) in der Sequenz, die die HBcAg-Aminosäurereste 75 bis 85 oder die entsprechende Sequenz des Kernproteins eines anderen Hepadnavirus kodiert, oder (b) zwei Restriktionsstellen hat, die diese Sequenz oder einen Teil dieser Sequenz flankieren, hat, derart, daß der Vektor an Restriktionsstelle (a) oder Restriktionsstelle (b) geschnitten wird;
(ii) Dephosphorylierung des aufgeschlossenen Vektors; und
(iii) Binden einer DNA-Sequenz, die eine fremde Aminosäuresequenz, welche ein Epitop enthält, kodiert, im Vektor.

10. Verfahren nach Anspruch 9, in dem die Restriktionsstelle (a) an den HBcAg-Kodons 80 und 81 oder an den entsprechenden Kernprotein-Kodons eines anderen Hepadnavirus auftritt.

11. Verfahren nach Anspruch 10, in dem der Expressionsvektor pPV-Nhe (wie in der Hinterlegung NCJMB 40210 verborgen ist) ist.

**Revendications**

**Revendications pour les Etats contractants suivants : BE, CH, DE, DK, FR, GB, IT, LI, NL, SE**

1. Particules composées d'une protéine chimérique d'antigène de core d'un hepadnavirus ayant une séquence d'acides aminés étrangère comprenant un épitope inséré dans ou remplaçant la totalité ou une partie de la séquence d'acides amines du résidu 75 au résidu 85 dans le cas où l'antigène de core est l'antigène de core de l'hépatite B (HBcAg) ou la séquence d'acides aminés correspondante dans le cas de l'antigène de core d'un autre hepadnavirus.

2. Particules selon la revendication 1, dans lesquelles la séquence d'acides aminés étrangère comporte jusqu'à 40 résidus d'acides aminés.

3. Particules selon la revendication 2, dans lesquelles la séquence d'acides aminés étrangère comporte jusqu'à 20 résidus d'acides aminés.

4. Particules selon l'une quelconque des revendications précédentes, dans lesquelles la séquence d'acides aminés étrangère est insérée entre les résidus 80 et 81 de HBcAg ou entre les résidus correspondants de la protéine de core d'un autre hepadnavirus.

5. Particules selon l'une quelconque des revendications précédentes, dans lesquelles l'épitope est l'épitope d'un pathogène.

6. Particules selon la revendication 5, dans lesquelles l'épitope est un épitope du virus de l'hépatite A, du virus de l'hépatite B, du virus de la grippe, du virus de la fièvre aphteuse, du poliovirus, de l'herpès simplex virus, du virus de la rage, du virus de la leucémie du chat, du virus de l'immunodéficience humaine de type 1 ou 2, du virus de l'immunodéficience du singe, du rhinovirus humain, du virus de la dengue ou du virus de a fièvre jaune.

7. Particules selon l'une quelconque des revendications précédentes, dans lesquelles une seconde séquence d'acides aminés étrangère telle que mentionnée est fusionnée à la partie N-terminale de la séquence d'acides aminés de la protéine de core.

8. Vecteur qui comprend une séquence d'ADN codant pour une protéine chimérique telle que spécifiée dans l'une quelconque des revendications précédentes et qui est capable, lorsqu'il est introduit dans un hôte approprié, d'exprimer la protéine chimérique.

9. Hôte transformé avec un vecteur selon la revendication 8, de sorte que la protéine chimérique puisse être exprimée dans celui-ci.

10. Procédé de préparation des particules telles que revendiquées dans l'une quelconque des revendications 1 à 7, procédé qui comprend les étapes consistant à cultiver un hôte selon la revendication 9 dans des conditions telles que la protéine chimérique soit exprimée dans celui-ci, et à récupérer les particules composées de la protéine chimérique qui sont ainsi formées.

11. Procédé de préparation d'un hôte tel que revendiqué à la revendication 9, procédé qui comprend la transformation d'un hôte avec un vecteur d'expression compatible selon la revendication 8.

12. Vecteur d'expression qui comprend une séquence d'ADN codant pour un antigène de core d'un hepadnavirus et ayant (a) un site de restriction dans la séquence codant pour les résidus d'acides aminés 75 à 85 de HBcAg ou la séquence correspondante de la protéine de core d'un autre hepadnavirus ou (b) deux sites de restriction flanquant une séquence telle que mentionnée ou une partie d'une séquence telle que mentionnée.

13. Vecteur selon la revendication 12, dans lequel le site de restriction (a) est situé aux codons 80 et 81 de HBcAg ou aux codons correspondants de la protéine de core d'un autre hepadnavirus.

14. Vecteur selon la revendication 12, qui est pPV-Nhe (tel que contenu dans le dépôt NCIMB 40210).

15. Procédé de préparation d'un vecteur tel que revendiqué à la revendication 8, procédé qui comprend les étapes consistant à :
(i) digérer avec la (les) endonucléase(s) de restriction appropriée(s) un vecteur d'expression tel que revendiqué dans l'une quelconque des revendications 12 à 14, de façon à couper le vecteur au site de restriction (a) ou au site de restriction (b);
(ii) déphosphoryler le vecteur digéré; et
(iii) ligaturer dans le vecteur une séquence d'ADN codant pour une séquence d'acides aminés étrangère comprenant un épitope.

16. Formulation pharmaceutique ou vétérinaire comprenant un véhicule ou un diluant acceptable d'un point de vue pharmaceutique ou vétérinaire et, comme ingrédient actif, des particules telles que revendiquées dans l'une quelconque des revendications 1 à 7.

**Revendications pour l'Etat contractant suivant : ES**

1. Procédé de préparation de particules composées d'une protéine chimérique d'antigène de core d'un hepadnavirus ayant une séquence d'acides aminés étrangère comprenant un épitope inséré dans ou remplaçant la totalité ou une partie de la séquence d'acides aminés du résidu 75 au résidu 85 dans le cas où l'antigène de core est l'antigène de core de l'hépatite B (HBcAg) ou la séquence d'acides aminés correspondante dans le cas de l'antigène de core d'un autre hepadnavirus, procédé qui comprend les étapes consistant à
   (I) cultiver un hôte transformé avec un vecteur d'expression compatible qui comprend une séquence d'ADN codant pour une protéine chimérique telle que mentionnée dans des conditions telles que la protéine chimérique soit exprimée dans l'hôte, et
   (II) récupérer les particules composées de protéine chimérique qui sont ainsi formées.

2. Procédé selon la revendication 1, dans lequel la séquence d'acides aminés étrangère comporte jusqu'à 40 résidus d'acides aminés.

3. Procédé selon la revendication 2, dans lequel la séquence d'acides aminés étrangère comporte jusqu'à 20 résidus d'acides aminés.

4. Procédé selon l'une quelconque des revendications précédentes, dans lequel la séquence d'acides aminés étrangère est insérée entre les résidus 80 et 81 de HBcAg ou entre les résidus correspondants de la protéine de core d'un autre hepadnavirus.

5. Procédé selon l'une quelconque des revendications précédentes, dans lequel l'épitope est l'épitope d'un pathogène.

6. Procédé selon la revendication 5, dans lequel l'épitope est un épitope du virus de l'hépatite A, du virus de l'hépatite B, du virus de la grippe, du virus de la fièvre aphteuse, du poliovirus, de l'herpès simplex virus, du virus de la rage, du virus de la leucémie du chat, du virus de l'immunodéficience humaine de type 1 ou 2, du virus de l'immunodéficience du singe, du rhinovirus humain, du virus de la dengue ou du virus de la fièvre jaune.

7. Procédé selon l'une quelconque des revendications précédentes, dans lequel une seconde séquence d'acides aminés étrangère telle que mentionnée est fusionnée à la partie N-terminale de la séquence d'acides aminés de la protéine de core.

8. Procédé de préparation d'un hôte transformé, procédé qui comprend la transformation d'un hôte avec un vecteur d'expression compatible qui comprend une séquence d'ADN codant pour une protéine chimérique d'antigène de core d'un hepadnavirus, tel que défini dans l'une quelconque des revendications précédentes.

9. Procédé de préparation d'un vecteur qui comprend une séquence d'ADN codant pour une protéine chimérique d'antigène de core d'un hepadnavirus tel que défini dans l'une quelconque des revendications 1 à 7 et qui est capable, lorsqu'il est fourni à un hôte approprié, d'exprimer la protéine chimérique; procédé qui comprend les étapes consistant à :
   (i) digérer avec la (les) endonucléase(s) de restriction appropriée(s) un vecteur d'expression qui comprend une séquence d'ADN codant pour un antigène de core d'un hepadnavirus et ayant (a) un site de restriction dans la séquence codant pour les résidus d'acides aminés 75 à 85 de HBcAg ou la séquence correspondante de la protéine de core d'un autre hepadnavirus ou (b) deux sites de restriction flanquant unee séquence telle que mentionnée ou une partie d'une séquence telle que mentionnée, de façon à couper le vecteur au site de restriction (a) ou au site de restriction (b) ;
   (ii) déphosphoryler le vecteur digéré; et
   (iii) ligaturer dans le vecteur une séquence d'ADN codant pour une séquence d'acides aminés étrangère comprenant un épitope.

10. Procédé selon la revendication 9, dans lequel le site de restriction (a) est situé aux codons 80 et 81 de HBcAg ou aux codons correspondants de la protéine de core d'un autre hepadnavirus.

**11.** Procédé selon la revendication 10, dans lequel le vecteur d'expression est pPV-Nhe (tel que contenu dans le dépôt NCIMB 40210).